# EUROPEAN PATENT SPECIFICATION

(11) **EP 0 557 790 B1**
(45) Date of publication and mention of the grant of the patent: **19.08.1998**
(21) Application number: 93102015.0
(22) Date of filing: 09.02.1993
(51) Int. Cl.: C07C 11/09, C07C 5/48, B01J 27/18, C07C 5/42

(54) **Process for producing isobutylene by oxidative dehydrogenation of isobutane**
Verfahren zur Herstellung von Isobutylen durch oxidative Dehydrierung von Isobutan
Procédé de fabrication d'isobutylène par déshydrogénation oxydante d'isobutane

(30) Priority: 28.02.1992 JP 41857/92; 12.03.1992 JP 53553/92
(43) Date of publication of application: 01.09.1993
(73) Proprietor: SUMITOMO CHEMICAL COMPANY LIMITED, Osaka-shi, Osaka 541 (JP); Takita, Yusaku, Oita-shi (JP)
(72) Inventor: Takita, Yusaku, Oita-shi (JP); Ui, Toshiaki, Niihama-shi (JP); Okusako, Kensen, Niihama-shi (JP); Miura, Naoki, Niihama-shi (JP); Nagai, Koichi, Niihama-shi (JP)
(74) Representative: Henkel, Feiler, Hänzel

(56) References cited:
- US-A- 3 851 009
- US-A- 3 873 633
- US-A- 4 218 343
- US-A- 4 497 971

## Description

The present invention relates to a process for producing isobutylene by oxidative dehydrogenation of isobutane. In more particular, it relates to a process for producing isobutylene by catalytic oxidative dehydrogenation with molecular oxygen in the presence of a specific catalyst in the gas phase.

Oxidative dehydrogenation is known as a method for producing industrially valuable unsaturated hydrocarbons from corresponding saturated hydrocarbons. For example, the following attempts are known to produce unsaturated hydrocarbons from saturated hydrocarbons having 3 or 4 carbon atoms by oxidative dehydrogenation.

Chemtech., March 1973, p. 186-189, describes examples of oxidative dehydrogenation of propane, n-butane or isobutane in the presence of a catalyst comprising potassium chloride, lithium chloride and manganese chloride supported on aluminum oxide.

European Patent No. 189,282 relates to oxidative dehydrogenation of ethane, propane or isobutane in the presence of a catalyst consisting essentially of tin oxide and phosphorus oxide. In the working examples, however, only a reaction of ethane to ethylene was reported.

U.S. Patent No. 4,751,342 describes the oxidative dehydrogenation of propane or n-butane in the presence of a catalyst comprising oxides of nickel, phosphorous and tin. Corresponding unsaturated hydrocarbons are obtained respectively.

German Patent No. 2,124,438 describes the oxidative dehydrogenation of isobutane to isobutylene using an alumina-supported metal oxide catalyst in the presence of hydrogen iodide. The conversion of isobutane was 28% and selectivity to isobutylene was 85%. The method, however, has the disadvantage of requiring the addition of hydrogen iodide.

Japanese Patent Kokai (Laid-open) No. 3-218327 discloses oxidative dehydrogenation of propane or isobutane in working examples using a catalyst comprising tin oxide and phosphorus oxide as the main component. It also discloses a catalyst comprising indium oxide and phosphorus oxide as the main components. However, the selectivity to propylene or isobutylene is low unless ammonia is added to the feed gas. In a reaction which uses only isobutane and oxygen, the conversion of isobutane was 1.4% and selectivity to isobutylene was 32%.

As described above, no catalytic system has yet been reported which can give a high selectivity to propylene, n-butylene or isobutylene in the oxidative dehydrogenation of propane, n-butane or isobutane. In the case of oxidative dehydrogenation of isobutane which uses no halogen nor ammonia, in particular, no example has been reported in which selectivity to isobutylene is higher than 50%.

US-A-3 851 009 discloses a process wherein organic compounds are dehydrogenated to compounds having a higher degree of unsaturation by contacting the feedstock in the vapor phase in the presence of an oxygen-containing gas with a catalyst comprising cobalt in association with iron, phosphorus and oxygen. Representative of such conversions is the oxidative dehydrogenation of isopentane to isoprene and isoamylenes. The conversion products are valuable compounds particularly useful as intermediates for the preparation of polymeric materials such as synthetic rubbers and the like.

US-A-4 218 343 discloses a process wherein hydrocarbon feedstocks are dehydrogenated to compounds having the same number of carbon atoms and a higher degree of unsaturation by contacting the feedstock in the vapor phase in the presence of molecular oxygen with a catalyst consisting essentially of nickel, lead, phosphorus and combined oxygen, and optionally, an alkali metal. Representative of such conversions is the oxidative dehydrogenation of butane to butenes and butadiene.

US-A-3 873 633 discloses a process wherein organic compounds are dehydrogenated to compounds having a higher degree of unsaturation by contacting the feedstock in the vapor phase in the presence of an oxygen-containing gas with a catalyst comprising cobalt, phosphorus, oxygen, and optionally, bismuth. Representative of such conversions is the oxidative dehydrogenation of butane to butadiene and isopentane to isoprene in the presence of a catalyst comprising bismuth-cobalt-phosphorus-oxygen and cobalt-phosphorus-oxygen, respectively. The conversion products are valuable compounds particularly useful as intermediates for the preparation of polymeric materials such as synthetic rubbers and the like.

US-A-4 497 971 discloses a process wherein a paraffin or mixture of paraffins having from 2 to 5 carbon atoms is oxidatively dehydrogenated in the presence of a cobalt based catalyst composition which has been calcined in the absence of oxygen. The catalyst composition comprises cobalt; phosphorus; at least one promoter selected from the group consisting of zinc, titanium, zirconium, niobium, indium, lead and bismuth; at least one alkali metal and oxygen. The catalyst composition may also contain sulfur and/or a halogen. If the feed to the oxidative dehydrogenation process contains paraffins having more than two carbon atoms, some cracking of such paraffins will also occur at the conditions at which the oxidative dehydrogenation process is carried out.

The object of the present invention is to provide a process for producing isobutylene with a high selectivity by performing the oxidative dehydrogenation of isobutane in the presence of a catalyst having a specific composition.

The present inventors have made extensive study to attain the above-mentioned object. As the result it has been found that a catalyst comprising phosphorus oxide and the oxide of one or more specific metals shows a high selectivity.

Thus, according to the present invention, there is provided a process for producing isobutylene by oxidative dehydrogenation of isobutane with molecular oxygen in the gas phase wherein the concentration of isobutane in the feed gas is 10-95% by volume, the molar ratio of isobutane to oxygen in the feed gas is 1 to 0.05-1, and the reaction temperature of the oxidative dehydrogenation is 300-600°C, which process uses a catalyst comprising a phosphorus oxide and an oxide of at least one element selected from the group consisting of zinc, nickel, iron, chromium, vanadium, manganese, cobalt, silver, copper, magnesium, lithium, scandium, titanium, gallium, yttrium, zirconium, antimony, lanthanum, cerium, samarium, ytterbium, hafnium and lead.

The catalyst used in the present invention can be prepared by using phosphoric acid or its salt and at least one metal compound (e.g., metal chloride, metal nitrate, metal sulfate, organometallic compound, metal oxide, ammonium salt of metal oxide and oxalate of metal oxide). The preparation of the catalyst can be performed by various methods known to the art, and is not particularly limited as to the method. For example, the complex oxide of vanadium and phosphorus is famous as a catalyst for producing maleic anhydride from n-butane, and various methods have been reported for its preparation.

The catalyst used in the present invention is composed of at least 2 components and may be in any of the following forms: a form of so-called complex oxide; a form wherein at least one component is supported on the oxide of the remaining components; and a form of a mixture of the oxides of respective components. The catalyst may also be used after supported on a carrier, such as silica, alumina, silicon carbide and silicon nitride, by conventional methods known to the art.

A feed gas used in the process of the present invention comprises mainly isobutane and oxygen. The feed gas may be diluted with an inert gas or the like. Gases which may be used for the dilution include nitrogen, helium, argon, carbon dioxide gas, water vapor, and further, lower alkanes such as methane, ethane propane and butane.

The source of oxygen used in the process of the present invention is not particularly limited. Usually, pure oxygen, oxygen-enriched air and air are employed. The molar ratio of isobutane to oxygen in the feed gas is 1 to 0.05-1. When the molar ratio of oxygen is too small, the reaction velocity tends to be low and the productivity per unit weight catalyst tends to be poor because the supply of oxygen is rate-determining in the reaction. When the molar ratio of oxygen is too large, on the contrary, the amount of oxidation byproducts other than isobutylene tends to increase, which lowers selectivity; further, sometimes a combustive reaction takes place and causes a problem in safety. Therefore, the molar ratio of isobutane to oxygen in the feed gas is 1 to 0.05-1.

The concentration of isobutane in the feed gas used in the process of the present invention is 10-95% by volume. However, when the concentration of isobutane is too low, the reaction velocity tends to be low, making the productivity per unit weight catalyst poor. The concentration of isobutane in the reaction material gas is 10-95% by volume.

The reaction temperature varies depending on the concentration of isobutane, its molar ratio in the feed gas, contact time, and other factors. It is usually in the range of 300°C to 600°C.

The reaction pressure is usually in the range of 1-50 bar, preferably 1-3 bar.

The process of the present invention may be performed by using any of the fixed bed, moving bed and fluidizing bed. When a fluidizing bed is used, the reaction may be performed in such a form that the oxidative dehydrogenation of isobutane is conducted by using a feed gas containing no oxygen and utilizing solely the oxygen present in the catalyst, and the catalyst is reoxidized with an oxygen-containing gas in a separate reactor.

The product obtained by the present reaction comprises mainly isobutylene. Slight amounts of propylene and methane are produced in addition thereto. Complete oxidation up to carbon oxides takes place only in a very low degree. Oxygen-containing compounds, such as metacrolein, are also formed in part.

The present invention is described in more detail below with reference to Examples, but it is in no way limited thereto.

### Example 1

A solution of 66.9 g of sodium secondary phosphate decahydrate (mfd. by Wako Pure Chemical Industries, Ltd.) in 2 ℓ of deionized water was added dropwise to a solution of 87.3 g of nickel nitrate hexahydrate in 1 ℓ of deionized water, to form a precipitate. The precipitate was washed with water and then filtered off with suction. Thus obtained filter cake was dried overnight at 130°C, then formed into granules of 16-32 meshes. The granules were roasted in a nitrogen stream at 500°C for 5 hours, whereby a catalyst was prepared.

Then, 1.4 g (1.9 mℓ) of the catalyst was placed into a glass reaction tube of 15 mm inner diameter. A gas mixture having a molar ratio of isobutane/oxygen/nitrogen of 75/10/15 was fed thereto at a flow rate of 30 mℓ/min while keeping the temperature at 450°C.

The analysis of the product gas showed that the conversion of isobutane was 9.3%, conversion of oxygen 89%, selectivity to isobutylene 63.1%, selectivity to propylene 11.4% and selectivity for metacrolein 4.9%.

### Example 2

The experimental procedures were essentially the same as described for Example 1 with the exception that the reaction temperature was changed from 450°C to 500°C.

The conversion of isobutane was 14.9%, conversion of oxygen 98.8%, selectivity to isobutylene 56.2%, selectivity to propylene 20.2% and selectivity to metacrolein 2.9%.

### Example 3

The experimental procedures were essentially the same as described for Example 1 with the exception that the reaction temperature was changed 450°C to 400°C.

The conversion of isobutane was 4.6%, conversion of oxygen 47.7%, selectivity to isobutylene 65.5%, selectivity to propylene 6.7% and selectivity to metacrolein 6.9%.

### Example 4

The experimental procedures were almost the same as described for Example 1 with the exception that the reaction temperature was changed 450°C to 500°C, and a gas mixture having a molar ratio of isobutane/oxygen/nitrogen to 75/5/20 was used.

The conversion of isobutane was 9.9%, conversion of oxygen 97.6%, selectivity to isobutylene 57.2%, selectivity to propylene 20.6% and selectivity for metacrolein 2.6%.

### Example 5

The experimental procedures were essentially the same as described for Example 4 with the exception that the reaction temperature was changed from 500°C to 450°C.

The conversion of isobutane was 5.2%, conversion of oxygen 89.0%, selectivity to isobutylene 64.5%, selectivity for propylene 11.7% and selectivity to metacrolein 5.9%.

### Example 6

Into an aqueous solution containing 27.8 g of hydroxylamine hydrochloride and 49.0 g of 80% phosphoric acid was dissolved 36.4 g of vanadium pentoxide to form a blue solution. The solution was evaporated to dryness, then dried overnight at 130°C, formed into granules of 16-32 meshes. The granules were roasted in a nitrogen stream at 500°C for 5 hours, whereby a catalyst was prepared. Thereafter, the same procedures described for Example 4 were followed except for using the catalyst prepared above.

The conversion of isobutane was 8.0%, conversion of oxygen 98.4%, selectivity to isobutylene 56.2%, selectivity to propylene 20.3%, and selectivity to metacrolein 0.5%.

### Examples 7-12

Catalysts were prepared in the same manner as described in Example 1 with the exception that 40.9 g of zinc chloride or 54.1 g of ferric chloride tetrahydrate was used in place of nickel nitrate hexahydrate.

The reaction operations were performed in the same manner as described in Example 1. The reaction temperatures were varied as indicated in Table 1. The results are shown in Table 1.

**Table 1**

| Example No. | Element | Reaction temp. (°C) | Conversion (%) | | Selectivity (%) | | |
|---|---|---|---|---|---|---|---|
| | | | Isobutane | Oxygen | Isobutylene | Propylene | Metacrolein |
| 7 | Zn | 450 | 9.4 | 85.8 | 62.2 | 11.3 | 4.7 |
| 8 | (II) | 500 | 16.6 | 98.4 | 55.5 | 20.0 | 3.1 |
| 9 | | 400 | 3.1 | 49.7 | 59.7 | 4.0 | 9.0 |
| 10 | Fe | 450 | 9.0 | 86.9 | 59.2 | 9.4 | 8.4 |
| 11 | (III) | 500 | 16.6 | 98.6 | 52.6 | 19.7 | 3.3 |
| 12 | | 550 | 21.5 | 99.1 | 48.7 | 23.6 | 2.3 |

### Examples 13-30

Catalysts were prepared in the same manner as described in Example 1 with the exception that metal chlorides or metal nitrates shown in Tables 2 and 3 were used in place of nickel nitrate hexahydrate. The metal chlorides or the metal nitrates were used in such amounts that an atomic ratio of metal to phosphorus gave 2 to 1 when the valency of the metal was one, 1 to 1 when the valency was two and 2 to 3 when the valency was three. The reaction operations were performed in the same manner as in Example 4. The reaction temperatures were varied indicated in Tables 2 and 3. The results are shown in Tables 2 and 3.

**Table 2**

| Example No. | Element | Reaction Temp. (°C) | Conversion (%) | | Selectivity (%) | | |
|---|---|---|---|---|---|---|---|
| | | | Isobutane | Oxygen | Isobutylene | Propylene | Metacrolein |
| 13 | Zn | 450 | 5.9 | 68.4 | 64.9 | 11.8 | 4.7 |
| 14 | (II) | 500 | 10.1 | 94.4 | 59.4 | 21.4 | 3.5 |
| 15 | Fe | 450 | 6.9 | 94.3 | 65.6 | 11.9 | 6.1 |
| 16 | (III) | 500 | 12.1 | 98.8 | 65.9 | 20.6 | 2.7 |
| 17 | Co | 450 | 6.5 | 81.7 | 67.8 | 12.3 | 6.2 |
| 18 | (II) | 500 | 11.1 | 98.3 | 58.8 | 21.1 | 4.3 |
| 19 | Mn | 450 | 4.1 | 60.2 | 67.7 | 12.3 | 6.0 |
| 20 | (II) | 500 | 9.7 | 97.3 | 59.7 | 21.5 | 5.9 |
| 21 | Cr | 450 | 7.4 | 97.9 | 63.8 | 11.6 | 6.5 |
| 22 | (III) | 500 | 12.1 | 98.2 | 57.6 | 20.7 | 2.7 |
| 23 | Cu | 450 | 2.9 | 98.5 | 71.1 | 1.8 | 4.1 |
| 24 | (II) | 500 | 7.1 | 98.3 | 71.6 | 6.6 | 1.7 |

**Table 3**

| Example No. | Element | Reaction Temp. (°C) | Conversion (%) | | Selectivity (%) | | |
|---|---|---|---|---|---|---|---|
| | | | Isobutane | Oxygen | Isobutylene | Propylene | Metacrolein |
| 25 | Ag | 450 | 4.2 | 76.2 | 78.3 | 2.7 | 3.0 |
| 26 | (I) | 500 | 7.8 | 98.0 | 68.7 | 9.4 | 2.3 |
| 27 | Mg | 450 | 5.2 | 95.8 | 74.8 | 3.7 | 5.7 |
| 28 | (II) | 500 | 8.9 | 98.6 | 69.3 | 9.0 | 1.8 |

### 1 Example 29

A 1.4 g portion of the catalyst of Example 19 (Example 20) was used, and a gas mixture having a molar ratio of isobutane/oxygen/nitrogen/water vapor of 18/9/55/18 was fed at a flow rate of 20 mℓ/min.

At a reaction temperature of 500°C, the conversion of isobutane was 14.8%, conversion of oxygen 57.1%, selectivity to isobutylene, 42.2%, selectivity to metacrolein 4.1% and selectivity to carbon monoxide 28.0%.

### Example 30

8.65 g of cerium nitrate hexahydrate (mfd. by Wako Pure Chemical Industries, Ltd.) was dissolved in 300 mℓ of deionized water. The solution was added to a solution of 2.65 g of pyrophosphoric acid (mfd. by Wako Pure Chemical Industries, Ltd.) in 50 mℓ of deionized water and mixed with stirring. A 25% aqueous ammonia solution was added to until the pH became 7, to form a precipitate. The precipitate was collected by filtration, washed with 500 mℓ of deionized water, then dried at 120°C, roasted in a nitrogen stream at 500°C for 4 hours and formed into granules of 10-24 meshes, whereby a catalyst was prepared.

3.0 g of the catalyst was diluted with 23.0 g of silicon carbide and placed into a glass reaction tube of 15 mm inner diameter. A gas mixture having a molar ratio of isobutane/oxygen/nitrogen of 75/5/20 was fed to the tube at a flow rate of 60 mℓ/min while keeping the temperature at 500°C.

The analysis of the product gas showed that the conversion of isobutane was 6.4%, conversion of oxygen 100%, selectivity to isobutylene 75.9%, selectivity to propylene 8.3% and selectivity to metacrolein 0.5%.

### Example 31

The experimental procedures were essentially the same as described in Example 30 with the exception that the reaction temperature was changed from 500°C to 450°C.

The conversion of isobutane was 4.5%, conversion of oxygen 100%, selectivity to isobutylene 75.4%, selectivity to propylene 5.7% and selectivity to metacrolein 1.1%.

### Example 32-36

Catalysts were prepared in the same manner as described in Example 30 with the exception that metal nitrates or metal nitrate hydrates shown in Table 4 were used in place of cerium nitrate hexahydrate. The metal nitrates or the metal nitrate hydrates were used in such amounts that an atomic ratio of metal to phosphorus gave 1 to 1 when the valency of the metal was two and 2 to 3 when the valency was three. The reaction operations were performed in the same manner as described in Example 30. The results are shown in Table 4.

**Table 4**

| Example No. | Element | Conversion (%) | | Selectivity (%) | | |
|---|---|---|---|---|---|---|
| | | Isobutane | Oxygen | Isobutylene | Propylene | Metacrolein |
| 32 | Sc(III) | 5.1 | 98 | 66.5 | 10.3 | 0.1 |
| 33 | Y (III) | 3.5 | 97 | 59.2 | 6.6 | 0.1 |
| 34 | Ga(III) | 2.3 | 80 | 58.9 | 13.7 | 1.6 |
| 35 | Sr(II) | 2.4 | 99 | 39.0 | 15.3 | 1.0 |
| 36 | Bi(III) | 1.7 | 95 | 32.6 | 13.5 | 1.2 |

### Example 37

Into 150 mℓ of deionized water heated to 50°C was added 21.3 g of lanthanum acetate sesquihydrate (mfd. by Nacalai Tesque Ltd.) to form a solution. Then, 8.5 g of pyrophosphoric acid dissolved in 50 mℓ of deionized water was added to the solution to form a white precipitate. The filtrate was evaporated to dryness with a rotary evaporator, roasted in a nitrogen stream at 500°C for 4 hours and then formed into granules of 10-24 meshes, whereby a catalyst was prepared.

The catalyst was used to perform the same reaction operations as described in Example 30. The conversion of isobutane was 4.4%, conversion of oxygen 93%, selectivity to isobutylene 59.2%, selectivity to propylene 19.0% and selectivity to metacrolein 2.3%.

### Examples 38 to 41

Catalysts were prepared in the same manner as described in Example 37 with the exception that metal acetates or metal acetate hydrates shown in Table 5 were used in place of lanthanum acetate sesquihydrate. The metal acetates or the metal acetate hydrates were used in such amounts that an atomic ratio of metal to phosphorus gave 2 to 1 when the valency of the metal was one, 1 to 1 when the valency was two and 2 to 3 when it was three. The reaction operations were performed in the same manner as in Example 30. The results are shown in Table 5.

**Table 5**

| Example No. | Element | Conversion (%) | | Selectivity (%) | | |
|---|---|---|---|---|---|---|
| | | Isobutane | Oxygen | Isobutylene | Propylene | Metacrolein |
| 38 | Sm(III) | 2.2 | 87 | 66.9 | 12.7 | 2.9 |
| 39 | Eu(III) | 2.2 | 99 | 33.9 | 8.7 | 0.4 |
| 40 | Cs(I) | 1.7 | 88 | 36.1 | 19.2 | 1.5 |
| 41 | Pb(II) | 1.1 | 63 | 58.1 | 15.3 | 2.8 |

### Example 42

To 300 mℓ of deionized water was added gradually 38.0 g of titanium tetrachloride (mfd. by Wako Pure Chemical Industries, Ltd.) to form a solution. Then 38.3 g of pyrophosphoric acid (mfd. by Wako Pure Chemical Industries, Ltd.) dissolved in 100 mℓ of deionized water was added to the solution to deposit a precipitate. After neutralization with a 25% aqueous ammonia solution, the precipitate was collected by filtration, washed with 1000 mℓ of deionized water, dried at 120°C. The precipitate was further roasted in a nitrogen stream at 500°C for 4 hours and formed into granules of 10-24 meshes, whereby a catalyst was prepared.

The catalyst was used to perform the same reaction operations as described in Example 30. The conversion of isobutane was 2.9%, conversion of oxygen 100%, selectivity to isobutylene 59.2%, selectivity to propylene 6.8% and selectivity to metacrolein 0.1%.

### Examples 43 to 45

Catalysts were prepared in the same manner as described in Example 42 with the exception that metal chlorides or metal chloride hydrates shown in Table 6 were used in place of titanium tetrachloride. The metal chlorides or the metal chloride hydrates were used in such amounts that an atomic ratio of metal to phosphorus gave 2 to 3 when the valency of the metal was three and 1 to 2 when the valency was four. The reaction operations were performed in the same manner as described in Example 30. The results are shown in Table 6.

**Table 6**

| Example No. | Element | Conversion (%) | | Selectivity (%) | | |
|---|---|---|---|---|---|---|
| | | Isobutane | Oxygen | Isobutylene | Propylene | Metacrolein |
| 43 | Hf(IV) | 3.0 | 99 | 54.9 | 5.6 | 0 |
| 44 | Yb(III) | 2.6 | 99 | 51.7 | 8.2 | 0 |
| 45 | Sb(III) | 1.0 | 80 | 60.6 | 16.1 | 3.2 |

### Example 46

To a solution of 177.2 g of zirconium oxychloride octahydrate (mfd. by Wako Pure Chemical Industries, Ltd.) in 1 ℓ of deionized water was added a solution of 113.8 g of sodium secondary phosphate decahydrate (mfd. by Wako Pure chemical Industries, Ltd.) dissolved at 50°C in 450 mℓ of deionized water, to form a white precipitate. The precipitate was collected by filtration, washed with 2 ℓ of deionized water, dried at 120°C, further roasted in a nitrogen stream at 500°C for 4 hours and formed into granules of 10-24 meshes, whereby a catalyst was prepared.

The catalyst was used to perform the same reaction operations as described in Example 30. The conversion of isobutane was 3.5%, conversion of oxygen 99%, selectivity for isobutylene 54.8%, selectivity to propylene 3.9% and selectivity to metacrolein 0%.

### Example 47

Lithium phosphate hemihydrate (mfd. by Katayama Kagaku Kogyo Co., Ltd.) was roasted in air at 500°C for 4 hours and then formed into tablets of 10-24 meshes to use as a catalyst.

The catalyst was used to perform the same reaction operations as described in Example 30. The conversion of isobutane was 2.4%, conversion of oxygen 83%, selectivity for isobutylene 57.2%, selectivity to propylene 7.1% and selectivity to metacrolein 2.7%.

## Claims

1. A process for producing isobutylene by oxidative dehydrogenation of isobutane with molecular oxygen in the gas phase wherein the concentration of isobutane in the feed gas is 10-95% by volume, the molar ratio of isobutane to oxygen in the feed gas is 1 to 0.05-1, and the reaction temperature of the oxidative dehydrogenation is 300-600°C, which process uses a catalyst comprising a phosphorus oxide and an oxide of at least one element selected from the group consisting of zinc, nickel, iron, chromium, vanadium, manganese, cobalt, silver, copper, magnesium, lithium, scandium, titanium, gallium, yttrium, zirconium, antimony, lanthanum, cerium, samarium, ytterbium, hafnium and lead.

## Patentansprüche

1. Verfahren zur Herstellung von Isobutylen durch oxidative Dehydrierung von Isobutan mit molekularem Sauerstoff in der Gasphase, wobei die Konzentration des Isobutans in dem gasförmigen Ausgangsmaterial 10 - 95 Vol.-%, das Molverhältnis Isobutan/Sauerstoff im gasförmigen Ausgangsmaterial 1/0,05 - 1 und die Reaktionstemperatur der oxidativen Dehydrierung 300 - 600°C betragen, wobei bei dem Verfahren ein Katalysator, umfassend ein Phosphoroxid und ein Oxid mindestens eines Elements, ausgewählt aus der Gruppe Zink, Nickel, Eisen, Chrom, Vanadium, Mangan, Kobalt, Silber, Kupfer, Magnesium, Lithium, Scandium, Titan, Gallium, Yttrium, Zirkon, Antimon, Lanthan, Cer, Samarium, Ytterbium, Hafnium und Blei, verwendet wird.

## Revendications

1. Procédé de préparation d'isobutylène par déshydrogénation oxydante d'isobutane à l'aide d'oxygène moléculaire en phase gazeuse, la concentration en isobutane dans le gaz d'alimentation étant de 10-95% en volume, le rapport molaire de l'isobutane à l'oxygène dans le gaz d'alimentation étant de 1 à 0,05-1, et la température de la réaction de déshydrogénation oxydante étant de 300-600°C, ledit procédé utilisant un catalyseur comprenant un oxyde de phosphore et un oxyde d'au moins un élément choisi dans le groupe constitué par le zinc, le nickel, le fer, le chrome, le vanadium, le manganèse, le cobalt, l'argent, le cuivre, le magnésium, le lithium, le scandium, le titane, le gallium, l'yttrium, le zirconium, l'antimoine, le lanthane, le cérium, le samarium, l'ytterbium, l'hafnium et le plomb.
